# EUROPEAN PATENT APPLICATION

(11) **EP 4 342 485 A1**
(43) Date of publication of application: **27.03.2024**
(21) Application number: 22197562.6
(22) Date of filing: 23.09.2022
(51) Int. Cl.: A61K 38/18, A61P 21/02, A61K 9/00

(54) **NGF FOR THE TREATMENT OF SPASTICITY**

(71) Applicant: Dompe' Farmaceutici S.P.A., 20122 Milan (IT)
(72) Inventor: ALLEGRETTI, Marcello, 67100 L'Aquila (IT); ARAMINI, Andrea, 67100 L'Aquila (IT); BRANDOLINI, Laura, 67100 L'Aquila (IT); CATTANI, Franca, 67100 L'Aquila (IT); MANTELLI, Flavio, 67100 L'Aquila (IT)
(74) Representative: Mauri, Elisabetta Maria Ester

(57) **Abstract**

The present invention relates to nerve growth factor (NGF) or a mutein thereof for use in the prevention or treatment of spasticity in a subject, wherein preferably said NGF or mutein is administered intranasally to said subject.

## Description

### FIELD OF THE INVENTION

The present invention relates to the prevention or treatment of spasticity in a subject.

### STATE OF THE ART

Spasticity is a motor disorder characterised by a velocity dependent increase in muscle tone, with exaggerated muscle tendon jerks that is caused by hyperexcitability of the muscle stretch reflex (J.W. Lance, "Symposium synopsis," in Spasticity: Disordered Motor Control, R. G. Feldman, R. R. Young, and W. P. Koella, Eds., pp. 485-494, 1980). It can arise as a consequence of traumatic insults, chronic neurodegenerative conditions or genetic defects that cause the disruption or malfunctioning of the areas of the brain and nerve tracks responsible for controlling the initiation and modulation of movements. These include, for example, cerebral palsy, stroke, traumatic brain injury, amyotrophic lateral sclerosis, primary lateral sclerosis, multiple sclerosis (MS) and hereditary spastic paraplegia (HSP) (Mathewson et al, Phys Med Rehabil Clin N Am 2015, 26(1): 57-67; Khundadze et al, Autophagy 2021, 17(11): 3690-3706; Chang et al, Crit Rev Phys Rehabil Med 2013, 25(1-2): 11-22).

Spasticity is highly invalidating condition for patients as it affects their ability to conduct basic daily activities such as hygiene, dressing, ambulation and sleep. Without therapy, in the long-term spasticity can lead to pain, permanent joint deformity, urinary tract infections, chronic constipation, peripheral neuropathy and pressure ulcers.

Therefore, treatment of this condition is imperative to improve the quality of life and avoid serious medical complication.

Currently available treatment options are limited and are aimed at only palliating the symptoms to allow individuals to live with the least amount possible of discomfort and restriction, without a significant number of side effects.

Available treatments include systemic medications and interventional procedures. Systemic medications comprise centrally acting agents, such as baclofen, anticonvulsants such as benzodiazepines and gabapentin and peripherally acting agents such as dantrolene. Interventional procedures include focal injections of botulinum toxin, phenol or alcohol, and the insertion of an intrathecal baclofen pump.

Unfortunately, all treatments available are characterized by adverse effect and risks which can outweigh the potential benefits they may provide. Systemic drugs may be better for patients with generalized spasticity but can cause a number of unwanted effects such as systemic muscle relaxation, sedation, fatigue and in some cases, produce tolerance and dependence.

Interventional therapies are generally associated with fewer systemic side effects if patients are compliant and the procedures are performed correctly. However, they need to be performed by an appropriately trained professional as they may result in serious complications in case of procedural errors, for example resulting from dissemination of the drug to other areas of the body.

It is therefore strongly felt the need of developing new effective, long lasting and safe therapeutic approaches for the prevention or treatment of spasticity. Furthermore, it is felt the need of identifying new treatments that address not only the symptoms of this conditions but also the underlying pathological mechanism.

### SUMMARY OF THE INVENTION

As it will be described in the experimental section, the present inventors have found that when NGF is administered intranasally, it is delivered at particularly high concentrations to the areas of the brain that are damaged in patients with spasticity.

The present inventors have also surprisingly found that the intranasal administration of NGF is effective in restoring the activity of motor neurons in these areas, thereby decreasing spasticity and improving motor ability of the patient.

Furthermore, the efficacy of intranasally administered NGF on spasticity is independent from the nature of induced damage. In fact, the inventors have found that NGF restores motor activity in animal models of both mechanically induced brain damage (TBI model) and in physiopathological damage of relevant neuronal cells (chemogenetic model).

On the basis of the data obtained, contrary to the available therapeutic options to date, NGF is therefore able not only to decrease the symptomatology of patients affected by spasticity, but to at least partially reverse the motor impairment associated thereof. Accordingly, object of the invention is NGF or a mutein thereof for use in the prevention or treatment of spasticity in a subject.

### BRIEF DESCRIPTION OF THE FIGURES

Figure 1 shows NGF biodistribution in rat brain areas after intranasal administration, measured by ELISA over 24 hours, as described in Example 1. Results are expressed as percent of the total amount absorbed over 24 hours in the rat brain after administration.
Figure 2 shows the "rotarod time latency" (% vs sham animals) in rats in which cholinergic neurons were chemogenetically blocked (ChAT off) or in sham rats (Sham), treated with vehicle (ChAT off and Sham, respectively) or with rhNGF by intranasal administration (ChAT off+NGF and Sham+NGF, respectively), as described in Example 2.

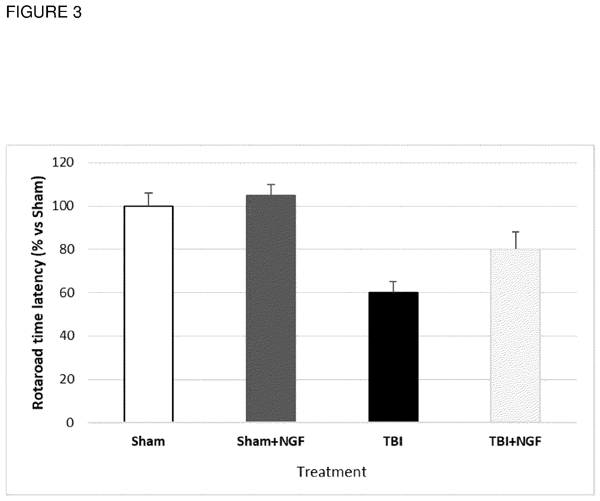

Figure 3 shows the "rotarod time latency" (% vs sham animals) in mice with Thraumatic Brain Injury (TBI) or sham (Sham), treated with vehicle or rhNGF (TBI+NGF and Sham+NGF, respectively) by intranasal administration, as described in Example 3.

### DETAILED DESCRIPTION OF THE INVENTION

A first object of the invention is nerve growth factor (NGF) or a mutein thereof for use in the prevention or treatment of spasticity in a subject.

Preferably, said NGF or mutein is administered intranasally to said subject.

Preferably, said spasticity is a spasticity caused by a condition selected from cerebral palsy, stroke, traumatic brain injury, amyotrophic lateral sclerosis, primary lateral sclerosis, multiple sclerosis (MS) and hereditary spastic paraplegia (HSP).

The terms "treatment" and "prevention" as used herein refer to the eradication/amelioration or prevention/delay in onset, respectively, of a disorder or of one or more of the symptoms associated thereof.

Preferably, said subject is a human subject.

According to one embodiment, said subject has been diagnosed with spasticity caused by a condition selected from cerebral palsy, stroke, traumatic brain injury, amyotrophic lateral sclerosis, primary lateral sclerosis, multiple sclerosis (MS) and hereditary spastic paraplegia (HSP) and said NGF or mutein thereof is for use in the treatment of said spasticity in said subject, preferably by intranasal administration to said subject. According to an alternative embodiment, said subject has been identified as being at risk of developing spasticity caused by a condition selected from cerebral palsy, stroke, traumatic brain injury, amyotrophic lateral sclerosis, primary lateral sclerosis, multiple sclerosis (MS) and hereditary spastic paraplegia (HSP) and said NGF or mutein thereof is for use in the prevention of said spasticity in of said subject prior to the development of said spasticity, preferably by intranasal administration to said subject.

Preferably said NGF is human NGF.

Preferably, said human NGF has the aminoacid sequence of SEQ ID NO:1 below SEQ. ID NO:1:

SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR

Alternatively, said human NGF has the aminoacid sequence of SEQ ID NO:2 below: SEQ ID NO:2:

SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA

Alternatively, said human NGF is a mixture of NGFs having sequences of SEQ ID NO:1 and SEQ ID NO:2.

The human NGF of SEQ ID NO:2 has an aminoacid sequence that only differ from the NGF of SEQ ID NO: 1 for the presence of two additional amminoacids at the C-terminus. Both forms of NGF are found in human cells and therefore are considered as wild type human NGF.

Therefore, when referring to "human NGF" or "wild type human NGF" in the present application, it is meant a human NGF of SEQ ID NO:1 or of SEQ ID NO:2.

Among the two forms of wild type NGF, the human NGF of SEQ ID NO:1 is particularly preferred. In fact, it has been found by the inventors that this specific form of NGF has particularly advantageous biological activity compared to the NGF of SEQ ID NO:2, since it has a higher neuroprotective activity on neuronal cells.

Preferably, said NGF is produced by recombinant DNA technology, preferably it is a human recombinant NGF (rhNGF). Methods of producing rhNGF are known to the person skilled in the art, for example those described in WO0022119A1 and WO2013092776A1. Preferably, said NGF has a purity higher than 70%, more preferably higher than 80%, higher than 90%, higher than 95%, higher than 98% or higher than 99%. The purity of NGF may be determined by conventional means known to those skilled in the art, for example by HPLC analysis.

The term "mutein of NGF" refers to a biologically active mutein of NGF, meaning a NGF protein having an aminoacid sequence with one or more aminoacid mutations, preferably substitutions, such that the therapeutic activity of wild type NGF is maintained. Preferably, said mutein is a mutein of wild type human NGF, as defined above.

Particularly preferred for use according to the invention, is a mutein of NGF, wherein the mutein is characterized by more than 80 %, more preferably more than 90 %, even more preferably more than 95%, and most preferably more than 98% sequence identity with wild type human NGF.

Preferably, the mutein is a mutein of wild type human NGF characterized by at least one mutation, preferably the aminoacid substitution of proline at position 61 by another amino acid, in the sequence of wild type human NGF. In a particularly preferred embodiment, proline at position 61 is substituted by serine.

Preferably, the mutein is a mutein of human NGF, characterized by at least one mutation of the amino acid sequence associated with reduced nociceptive activity. More preferably said mutein is characterized by at least one mutation, preferably amino acid substitution, at any of positions 95-101 of wild type human NGF. Even more preferably, said mutein is characterized by the substitution of the arginine in position 100 of wild type human NGF by another amino acid. More preferably, arginine at position 100 of wild type human NGF is substituted by glutamic acid.

Preferably, the mutein is a mutein of wild type human NGF characterized by at least the aminoacid substitution of proline at position 61 by another amino acid, preferably serine, and the substitution of the arginine in position 100 of wild type human NGF by another amino acid, preferably glutamic acid.

Particularly preferred muteins according to the invention have the aminoacid sequences of SEQ ID NO:3-6 below
SEQ ID NO:3: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VR
SEQ ID NO:4: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VRRA
SEQ ID NO:5: 
SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VR
SEQ ID NO:6:

SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VRRA

The above described muteins are particularly advantageous for use according to the invention since they maintain the same bioactivity than wild type human NGF but are able to induce a lower nociceptive sensitivity compared to the corresponding wild type human NGF. Preferably, said mutein of human NGF is produced by recombinant DNA technology. Methods of producing muteins of rhNGF according to the invention by recombinant DNA technology are known to the person skilled in the art, for example those described in WO2019/207106.

Preferably, the NGF or mutein for use according to the invention is administered to the subject from one to three times a day for a period of treatment of between 7 and 300 days, preferably between 60 and 240 days, more preferably between 100 and 200 days. Several cycles of treatment may be performed.

Preferably, when NGF is administered intranasally, the amount of NGF or mutein thereof per each administration is between 5 µg and 1 mg, more preferably between 10 µg and 400 µg, even more preferably between 15 µg and 200 µg.

The effective amount of said NGF or mutein used in each administration, the duration of the treatment and the number of administrations for day are selected by the skilled person on the basis of the characteristics of the subject to be treated, the severity of the spasticity and on the basis of assessment tests carried out during the treatment.

A further object of the present invention relates to a pharmaceutical composition for intranasal administration comprising the NGF or mutein as described above and at least one pharmaceutically acceptable excipient suitable for intranasal use.

Preferably, the pharmaceutical composition for intranasal administration of the invention is a liquid intranasal composition.

Preferably, the pharmaceutical composition according to the present invention comprises an effective amount of the NGF or mutein as described above and at least one pharmaceutically acceptable excipient suitable for intranasal use, preferably selected from solvents, thickening agents, mucoadhesive agents, buffers, antioxidants, preservatives, and penetration enhancers.

Preferably, the concentration of said NGF or mutein in the liquid intranasal composition according to the invention is between 5 µg/ml and 1 mg/ml, more preferably between 10 µg/ml and 400 µg/ml, even more preferably between 15 µg/ml and 200 µg/ml. Preferably, said solvent is water.

Preferably, said mucoadhesive agent is glycerol, more preferably at a concentration between 0.05 % w/v and 0.2% w/v, more preferably of 0.1% w/v.

Preferably, said antioxidant is methionine, more preferably at a concentration between 0.005 mg/ml and 0.02 mg/ml, more preferably of 0.01 mg/ml.

Preferably said surfactant is Kolliphor P188, more preferably at a concentration between 0.05 % w/v and 0.2% w/v, more preferably of 0.1% w/v.

Preferably, said buffer is phosphate buffer.

Preferably, said penetration enhancer is n-Dodecyl-β-D-maltoside, more preferably at a concentration between 0.1 % w/v and 1% w/v, more preferably of 0.5% w/v.

A particularly preferred liquid intranasal composition according to the invention comprises, preferably consists of, said NGF or mutein, sodium chloride, phosphate buffer and water.

Another particularly preferred liquid intranasal composition according to the invention comprises, preferably consists of, said NGF or mutein as described above, sodium chloride, phosphate buffer, Kolliphor P188, L-Methionine, and water.

Another particularly preferred liquid intranasal composition according to the invention comprises, preferably consists of said NGF or mutein, sodium chloride, phosphate buffer, Kolliphor P188, L-Methionine, Glycerol, n-Dodecyl-β-D-maltoside and water. Preferably, the liquid intranasal composition according to the invention comprises, preferably consists of, the following components:
- NGF or a mutein thereof, as described above, preferably at a concentration between 0.3 and 2 mg/ml, more preferably between 0.5 and 1.5 mg/ml,
- NaH2PO4 * H2O, preferably at a concentration between 5 and 8 mg/ml, more preferably of 6.9 mg/mL,
- NaCl, preferably at a concentration between 5 and 6.5 mg/ml, more preferably of 5.84 mg/mL,
- Kolliphor P188, preferably at a concentration between 0.05 % w/v and 0.2% w/v, more preferably of 0.1% w/v,
- L-Methionine, preferably at a concentration between 0.05 mg/ml and 0.2 mg/ml, more preferably of 0.1 mg/ml,
- Optionally, n-Dodecyl-β-D-maltoside, preferably at a concentration between 0.1 % w/v and 1% w/v, more preferably of 0.5% w/v, and/or glycerol, preferably at a concentration between 0.05 % w/v and 0.2% w/v, more preferably of 0.1% w/v,
- Water.

The pharmaceutical composition according to the invention may be suitably formulated using appropriate methods known in the art or by the method disclosed in Remington's Pharmaceutical Science (recent edition), Mack Publishing Company, Easton Pa.

In a further aspect, the present invention relates to a method for the prevention or treatment of spasticity in a subject. Preferably, said method comprises intranasally administering to the subject NGF or a mutein thereof, as described above, in a therapeutically effective amount.

Preferably, in the method according to the invention, said NGF or mutein is administered as described above.

Preferably, said NGF or mutein used in the method of the invention is in form of a pharmaceutical composition, as above described.

The invention will be further described in the following examples, which do not limit the scope of the invention described in the claims.

### EXPERIMENTAL SECTION

### Example 1

The biodistribution of NGF in rat brain areas after single intranasal administration was evaluated.

A formulation containing 1.2 mg/mL of rhNGF was administered once intranasally to rats. Rats were sacrificed at different time-points corresponding to 2 hours, 4 hours, 8 hours and 24 hours after treatment.

Samples from parietal cortex, hypothalamus, thalamus, striatum, hippocampus, brainstem, frontal cortex and medial septum were collected for rhNGF quantitative determination by ELISA and cumulative absorption (2-24 hours) was calculated.

Before analysis, brain samples were homogenized in ice by ultraturrax and centrifuged for supernatants recovery. A commercial ELISA kit (RayBiotech, Catalogue ELH-BNGF) was used according to the instructions provided by the supplier. The calibration curve range for NGF determination was established at 20,5 - 5000 pg/mL.

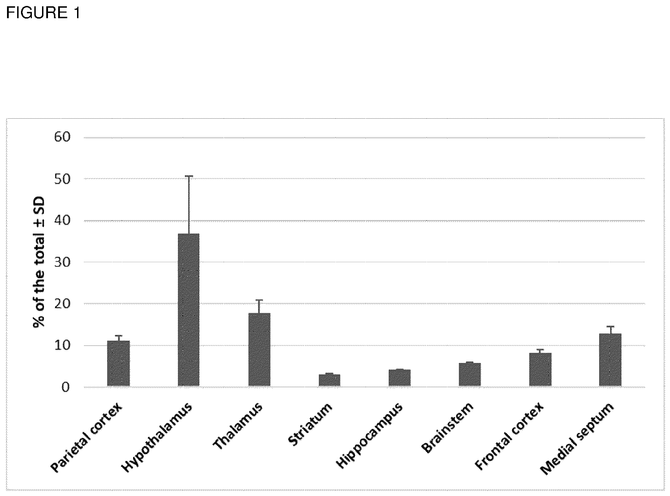

As shown in Figure 1, NGF absorption was evident in all brain tissues, but the protein particularly concentrates in areas deputed to motor activity, especially in hypothalamus and thalamus.

### Example 2

The role of NGF in counteracting the spasticity and associated loss of balance and motor coordination was evaluated in a rat chemogenetic model representative of neurons defects observed in HSP. Briefly, the selective blockade of cholinergic striatal neurons was achieved by stereologically injecting Cre-dependent adeno-associated virus (AAV-hSynDIO-hM4Di-mCherry) into relevant brain areas and the neuronal deactivation was achieved by clozapine-N-oxide (CNO) treatment acting on the specific promoter used to selectively modulate the functionality of cholinergic striatal neurons.

In this experiment the selective contribution of cholinergic neurons in the development of motor dysfunctions was evaluated by rotaroad test. In addition, the functional relevance of a possible recovery in cortico-striatal connectivity promoted by intranasal administration of rhNGF (50µg/kg for 3 days) was demonstrated.

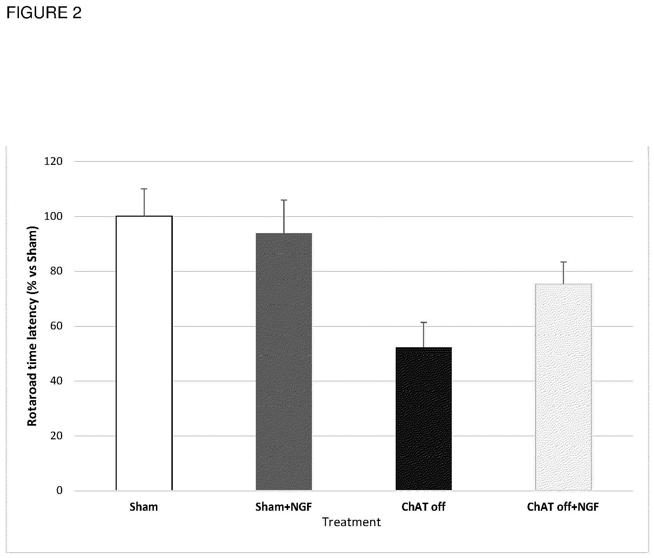

As reported in the Figure 2, the blockade of cholinergic neurons (ChAT off) reduced the rotarod latency time at about the 50% respect to the sham animals, whereas the treatment with NGF (ChAT off + NGF) restored the motor balance at about the 75% respect to the sham. No relevant mobility changes were observed in sham animals treated with NGF. Measurements were performed 24 hours after the last treatment.

### Example 3

The effect of NGF was also evaluated in a model of TBI in mice. Male C57BL/6 mice (Envigo, Italy) weighing 18-20 g were lightly anesthetized with isoflurane and placed in a prone position on a spongy support. The head were not fixed. To establish the TBI, a weight of 120 g was dropped from a height of 25 cm on mice skulls. The extent of the weight is needed to induce a consistent injury in the brain able to establish a severe motor dysfunction. Sham mice were submitted to the same procedure as described for TBI, but without release of the weight.

Treatment with NGF (50µg/kg/day) started when spasticity symptoms were well established.

The entity of damage generated, as well as the NGF ability to restore the muscle functionality after 3 days treatment (50µg/kg/day), was evaluated as motor coordination impairments by the rotarod test. The test consisted in two session of walking on a rotating cylinder, separated by a 1-hour pause. After a 30-seconds adaptation, rotation speed is gradually increased from 3 to 30 rpm, for maximum test duration of 5 minutes. Latency to fall (seconds) was recorded and final data were expressed as percentage of time latency versus sham.. As reported in Figure 2, with respect to the sham performance, the TBI reduced the rotarod latency time at about the 60%, whereas the treatment with NGF restored the motor balance at about the 80%. No effect was observed of NGF per se in the treatment of sham animals. Measurements were performed 24 hours after the last treatment.

## Claims

1. Nerve growth factor (NGF) or a mutein thereof for use in the prevention or treatment of spasticity in a subject.

2. Nerve growth factor (NGF) or a mutein thereof for use as claimed in claim 1, wherein said NGF is administered intranasally to said subject.

3. NGF or mutein for use as claimed in claim 1 or 2, wherein said spasticity is caused by a condition selected from cerebral palsy, stroke, traumatic brain injury, amyotrophic lateral sclerosis, primary lateral sclerosis, multiple sclerosis (MS) and hereditary spastic paraplegia (HSP).

4. NGF or mutein for use as claimed in claims 1 to 3, wherein said NGF is human NGF, more preferably it is recombinant human NGF.

5. NGF or mutein for use as claimed in claim 4, wherein said human NGF has the aminoacid sequence of SEQ ID NO:1.

6. Mutein for use as claimed in claim 4 or 5, having the aminoacid sequence of SEQ ID NO:3, SEQ ID NO:4, SEQ ID NO:5 or SEQ ID NO:6.

7. NGF or mutein for use as claimed in claims 1 to 6, wherein said NGF or mutein is administered from one to three times a day for a period of treatment of between 7 and 300 days, preferably between 60 and 240 days, more preferably between 100 and 200 days.

8. NGF or mutein for use as claimed in claims 1 to 6, wherein said NGF is administered intranasally and the amount of NGF per each administration is between 5 µg and 1 mg, more preferably between 10 µg and 400 µg, even more preferably between 15 µg and 200 µg.

9. A pharmaceutical composition for intranasal administration comprising NGF and at least one pharmaceutically acceptable excipient, for use in the prevention or treatment of spasticity in a subject.

10. A pharmaceutical composition for use as claimed in claim 9, wherein said spasticity is caused by a condition selected from cerebral palsy, stroke, traumatic brain injury, amyotrophic lateral sclerosis, primary lateral sclerosis, multiple sclerosis (MS) and hereditary spastic paraplegia (HSP).

11. A pharmaceutical composition for use as claimed in claim 9 or 10, wherein said NGF or mutein thereof is present in the composition at a concentration between 5 µg/ml and 1 mg/ml, more preferably between 10 µg/ml and 400 µg/ml, even more preferably between 15 µg/ml and 200 µg/ml.

12. A pharmaceutical composition for use as claimed in claims 9 to 11, comprising, preferably consisting of NGF or mutein thereof, sodium chloride, phosphate buffer and water.

13. A pharmaceutical composition for use as claimed in claims 9 to 12, wherein said NGF is human NGF, more preferably it is recombinant human NGF.

14. A pharmaceutical composition for use as claimed in claim 13, wherein said human NGF has the aminoacid sequence of SEQ ID NO:1 or SEQ ID NO:2.

15. A pharmaceutical composition for use as claimed in claim 13, wherein said mutein has the aminoacid sequence of SEQ ID NO:3, SEQ ID NO:4, SEQ ID NO:5 or SEQ ID NO:6.
